# EUROPÄISCHE PATENTSCHRIFT

(11) **EP 1 744 158 B1**
(45) Veröffentlichungstag und Bekanntmachung des Hinweises auf die Patenterteilung: **18.06.2008**
(21) Anmeldenummer: 05015489.7
(22) Anmeldetag: 16.07.2005
(51) Int. Cl.: G01N 33/18, C02F 3/00

(54) **Verfahren zur Bestimmung der metabolischen Stabilität von Schlamm, insbesondere von Klärschlamm**
Method for determining the metabolic stability of sludge
Procédé pour déterminer la stabilité métabolique d'une boue

(43) Veröffentlichungstag der Anmeldung: 17.01.2007
(73) Patentinhaber: Pauly, Udo, Dr., 37133 Friedland (DE)
(72) Erfinder: Pauly, Udo, Dr., 37133 Friedland (DE); Rehfus, Stefan, 37085 Göttingen (DE)
(74) Vertreter: Rehberg Hüppe + Partner

(56) Entgegenhaltungen:
- EP-A- 1 466 869
- EP-A- 1 486 466
- PATENT ABSTRACTS OF JAPAN Bd. 013, Nr. 326 (C-620), 24. Juli 1989 (1989-07-24) & JP 01 104395 A (HITACHI PLANT ENG & CONSTR CO LTD), 21. April 1989 (1989-04-21)

## Beschreibung

Die Erfindung betrifft ein Verfahren zur Bestimmung der metabolischen Stabilität von Schlamm, insbesondere Klärschlamm, wobei eine Sauerstoffzehrung des Schlamms nach Unterbrechung einer Sauerstoffzufuhr zu dem Schlamm beobachtet wird.

Eine ausreichende Stabilisierung von Klärschlamm ist beispielsweise Voraussetzung für dessen Vererdung in mit Pflanzen, wie beispielsweise Schilfpflanzen, bewachsenen Vererdungsbecken. Bei nicht ausreichender Stabilisierung des Klärschlamms wird der Pflanzenbestand in dem Vererdungsbecken und damit die kontinuierliche Funktion des Vererdungsbeckens gefährdet.

Ein bekanntes Hauptkriterium für die Beurteilung der metabolischen Stabilität von Schlamm, insbesondere von Klärschlamm, ist dessen Sauerstoffzehrung. Wenn die Sauerstoffzehrung einen Grenzwert von typischerweise 60 g Sauerstoff pro 1 kg Trockenrückstand am Tag bzw. 100 g Sauerstoff pro 1 kg organischem Trockenrückstand am Tag einhält, kann von einem weitgehend stabilisierten Schlamm ausgegangen werden.

### STAND DER TECHNIK

Aus der DE 44 42 002 C2 ist ein Verfahren der eingangs beschriebenen Art bekannt, bei dem unmittelbar in einem Reaktor zur oxidativen Behandlung von Abwasser nach einer oxidativen Phase mit Hilfe eines Sauerstoffmessgeräts über die Abnahme des Sauerstoffgehalts der aktuelle biochemische Sauerstoffbedarf erfasst und damit unmittelbar im Prozess, bevor das Abwasser den Reaktor verlässt, überprüft wird, ob das Ende der Behandlung erreicht ist, oder weiterbehandelt werden muss. Mit den bekannten Verfahren ist jedoch weder ermittelbar, in welchem Umfang das Abwasser noch weiter behandelt werden muss, noch welches Potential bei einer Weiterbehandlung des Abwassers in Bezug auf eine weitergehende Stabilisierung vorhanden wäre. Es wird ausschließlich der derzeitige Zustand des Abwassers in Bezug auf seine Sauerstoffzehrung bestimmt.

Ein weiteres Verfahren der eingangs beschriebenen Art ist aus der EP 1 466 869 A1 bekannt. Hier wird die Abnahmegeschwindigkeit des Sauerstoffs in einem Belebungsbecken, nachdem die Sauerstoffzufuhr unterbrochen wurde, dazu benutzt, die Zeitdauer zu berechnen, während welcher dem Belebungsbecken noch Sauerstoff zugeführt werden muss. Im Einzelnen wird dazu der Betrieb des Belebungsbeckens mit einer Belüftungsvorrichtung in eine Messphase und in eine Regelphase aufgeteilt. Die Messphase ihrerseits teilt sich in eine erste und eine zweite Phase auf. Während der ersten Phase wird die Belüftungsvorrichtung so lange betrieben, bis eine erste Sauerstoffkonzentration erreicht ist. Anschließend wird die Benutzungsvorrichtung abgeschaltet und während der zweiten Phase nicht betrieben, so dass die Sauerstoffkonzentration auf eine zweite Konzentration absinkt, die geringer ist als die erste Sauerstoffkonzentration. Aus den Messwerten der Sauerstoffkonzentration wird die Abnahmegeschwindigkeit während des Sauerstoffs während der zweiten Phase berechnet. Ausgehend von dieser Sauerstoff-Abnahmegeschwindigkeit wird nun die Zeitdauer berechnet, während welcher in der anschließenden Regelphase die Belüftungsvorrichtung betrieben werden soll. In der Regelphase wird dann die Belüftungsvorrichtung während des errechneten Zeitraums betrieben. Mehrere Abfolgen von Mess- und anschließender Regelphase und ggf. Belüftungsphase können sich wiederholen. Während jeder Messphase wird erneut die Sauerstoff-Abnahmegeschwindigkeit bestimmt, und entsprechend den jeweils vorgefundenen Bedingungen wird die Belüftung in der sich anschließenden Regelphase durchgeführt.

In dem Verfahren nach JP01104395 wird hingegen eine Schlammprobe entnommen. Diese wird in einem Probenbehälter zunächst belüftet. Nach Beendigung der Belüftung wird die Abnahme der Gelöstsauerstoffkonzentration mit einem Sauerstoffsensor gemessen.

In der Beschreibungseinleitung der EP 0 414 182 B1 ist ein manometrisches Verfahren nach Warburg in einer modifizierten Form beschrieben, wie es mit einem Produkt Sapromat® der Firma Voith durchführbar ist. Der Sapromat® umfasst ein Reaktionsgefäß, einen Sauerstofferzeuger und einen Druckindikator, die miteinander verbunden und in einem temperierten Wasserbad untergebracht sind. Eine auf ihre Sauerstoffzehrung hin zu untersuchende Probe wird in dem Reaktionsgefäß durch ein Magnetrührer umgewälzt, so dass die von ihr aufgezehrte Sauerstoffmenge aus dem darüber liegenden Gasraum in die Probe eintreten kann. Durch einen Gaswechsel aus dem Sauerstoffeintrag in die Probe, der Kohlendioxydproduktion in der Probe und sich anschließender Kohlendioxydabsorption entsteht ein Unterdruck, auf den der Druckindikator anspricht und bis zum Druckausgleich den Sauerstofferzeuger in Gang setzt. Der dabei für die Sauerstofferzeugung im Sauerstofferzeuger aufgewandte Elektrolysestrom wird als Maß für die von der Probe verbrauchte Sauerstoffmenge verwendet. Das bekannte Verfahren bedarf mit dem Sapromat® eines druckdichten Aufbaus, der seine Durchführung aufwändig macht. Auch die Sauerstoffzufuhr zu der Probe in Form von elektrolytisch gewonnenem Sauerstoff ist aufwändig.

### AUFGABE DER ERFINDUNG

Der Erfindung liegt die Aufgabe zugrunde, ein Verfahren der eingangs beschriebenen Art aufzuzeigen, mit dem mit geringem apparativen Aufwand die metabolische Stabilität von Schlamm so erfassbar sind, dass Aussagen darüber getroffen werden können, in welchem Umfang eine etwaige Nachbehandlung des Schlamms erforderlich ist und welches Potential in Bezug auf die metabolische Stabilität eine solche Nachbehandlung hätte.

### LÖSUNG

Die Aufgabe der Erfindung wird durch ein Verfahren mit den Merkmalen des Patentanspruchs 1 gelöst. Bevorzugte Ausführungsformen des neuen Verfahrens sind in den Unteransprüchen 2 bis 9 beschrieben.

### BESCHREIBUNG DER ERFINDUNG

Bei dem, neuen Verfahren wird die Sauerstoffzufuhr zu einer Probe des Schlamms in zeitlichem Abstand mindestens zweimal hintereinander unterbrochen. Mindestens einmal während jeder Unterbrechung der Sauerstoffzufuhr wird in gleichem Abstand zu dem Beginn der Unterbrechung der Sauerstoffzufuhr ein Sauerstoffgehalt der Probe ermittelt. Die metabolische Stabilität wird unter Berücksichtigung der Änderung des nach jeder Unterbrechung der Sauerstoffzufuhr ermittelten Sauerstoffgehalts bestimmt.

Bei dem neuen Verfahren wird nicht nur über die Bestimmung eines Werts für den Sauerstoffgehalt in der Probe, auf den der Sauerstoffgehalt in der Probe nach der Unterbrechung der Sauerstoffzufuhr in bestimmter Zeit abgefallen ist, die aktuelle Sauerstoffzehrung des Schlamms, von dem die Probe stammt, bestimmt. Zusätzlich wird das Entwicklungspotential des Schlamms für den Fall zusätzlicher Sauerstoffzufuhr erfasst, indem die Sauerstoffzehrung mindestens siebenmal hintereinander unter zwischenzeitlicher Zufuhr von Sauerstoff zu der Probe ermittelt und damit Änderungen der Sauerstoffzehrung aufgrund der zusätzlich erfolgten Sauerstoffzufuhr erfasst werden. Dennoch ist das neue Verfahren in sehr einfacher Weise durchführbar und erfordert nur einen minimalen apparativen Aufwand, wie sich aus der nachstehenden Beschreibung seiner bevorzugten Ausführungsformen und auch der sich anschließenden Beschreibung eines konkreten Ausführungsbeispiels ergeben wird.

Um die potentielle Entwicklung des Schlamms aufgrund von Sauerstoffzufuhr möglichst vollständig zu erfassen wird bei dem neuen Verfahren die Sauerstoffzufuhr zu der Probe in zeitlich gleichen Abständen einige Male hintereinander unterbrochen. Konkret kann die Sauerstoffzufuhr zu der Probe beispielsweise zwanzig Mal hintereinander unterbrochen werden. Die Anzahl der sinnvollen Unterbrechungen hängt natürlich auch davon ab, wie viel Sauerstoff der Probe zwischenzeitlich zugeführt wird. Um eine auch unter Berücksichtigung von Fehlerstatistik aussagekräftige Messreihe zu erhalten, wird die Sauerstoffzufuhr zu der Probe mindestens sieben Mal hintereinander unterbrochen.

Um genauere Aussagen über die Sauerstoffzehrung der Probe nach jeder Unterbrechung der Sauerstoffzufuhr machen zu können, ist es sinnvoll, in gleichen Abständen von dem Beginn der Unterbrechung der Sauerstoffzufuhr den Sauerstoffgehalt der Probe mehrfach zu ermitteln und den Verlauf des Sauerstoffgehalts während jeder Unterbrechung der Probe zu beobachten. Aus dem Verlauf des Sauerstoffgehalts in der Probe während jeder Unterbrechung der Sauerstoffzufuhr lässt sich die Sauerstoffzehrung der Probe deutlich genauer bestimmen als aus nur einem einzigen Messwert. Neben einer Erfassung des Sauerstoffgehalts in der Probe in gleichen zeitlichen Abständen kann der Sauerstoffgehalt in der Probe auch kontinuierlich bestimmt werden, um seinen Verlauf während jeder Unterbrechung der Sauerstoffzufuhr zu beobachten und auszuwerten.

Weiterhin ist es sinnvoll, mindestens einmal vor jeder Unterbrechung der Sauerstoffzufuhr den Sauerstoffgehalt der Probe zu ermitteln. Hierdurch wird nicht nur ein Ausgangswert für die anschließend betrachtete Sauerstoffzehrung bei Unterbrechung der Sauerstoffzufuhr gewonnen, sondern es wird auch überwacht, dass die Sauerstoffzufuhr den Sauerstoffgehalt in der Probe auf ein gewünschtes Mindestmaß, welches für die Betrachtung der Sauerstoffzehrung in der Probe notwendig ist, anhebt.

Wenn vor jeder Unterbrechung der Sauerstoffzufuhr in gleichen Abständen von dem Beginn der Sauerstoffzufuhr der Sauerstoffgehalt der Probe mehrfach ermittelt und der Verlauf des Sauerstoffgehalts während der Sauerstoffzufuhr beobachtet wird, kann die Sauerstoffzufuhr zu der Probe zwischen ihren Unterbrechungen noch besser überwacht werden. Außerdem ist es möglich, aus dem Verlauf des Wiederanstiegs des Sauerstoffgehalts in der Probe, ebenfalls Rückschlüsse auf die Sauerstoffzehrung der Probe zu ziehen. Natürlich kann auch die Ermittlung des Sauerstoffgehalts der Probe während der Sauerstoffzufuhr wahlweise kontinuierlich erfolgen.

Als für die Bestimmung der metabolischen Stabilität von Schlamm, insbesondere Klärschlamm, geeignete Intervalle haben sich eine Sauerstoffzufuhr für jeweils mindestens 30 min., beispielsweise 45 min., und eine Unterbrechung der Sauerstoffzufuhr für mindestens jeweils 10 min., beispielsweise jeweils 15 min., herausgestellt. Wenn die beiden Beispielswerte einer Sauerstoffzufuhr für jeweils 45 min. und einer Unterbrechung der Sauerstoffzufuhr für jeweils 15 min. miteinander kombiniert werden, beträgt die Dauer eines Messzyklus aus Sauerstoffzufuhr und anschließender Unterbrechung der Sauerstoffzufuhr jeweils 1 h. Wenn 24 dieser Messzyklen hintereinander durchgeführt werden, wird die Probe des Schlamms einen Tag lang gemessen. Dies entspricht einer bevorzugten Ausführungsform des neuen Verfahrens.

Es wurde bereits angedeutet, dass es wichtig ist, dass die Sauerstoffzufuhr zu der Probe von ihrem Umfang her ausreichend groß ist. Definierte Bedingungen ergeben sich dann, wenn die Sauerstoffzufuhr den Sauerstoffgehalt der Probe immer bis auf ihre Sättigungskonzentration, die bei ≥ 6 mg Sauerstoff in 1 Liter Probe liegt, anhebt. Sich darüber bewegende Schwankungen der Sauerstoffzufuhr haben auf die Menge des der Probe tatsächlich zur Verfügung gestellten Sauerstoffs keinen Einfluss. Entsprechend kann bei dem neuen Verfahren die Sauerstoffzufuhr mit sehr einfachen Mitteln durchgeführt werden.

Konkret kann die Sauerstoffzufuhr zu der Probe durch Einblasen von Druckluft in die Probe bewirkt werden. Dies kann mit einer einfachen Aquariumpumpe realisiert werden, mit der üblicherweise Umgebungsluft in ein Aquarium eingeblasen wird, um die Fische in dem Aquarium mit Sauerstoff zu versorgen.

Es versteht sich, dass die Sauerstoffzufuhr zu der Probe das gesamte Volumen der Probe homogen erfassen sollte. Dies kann einfach dadurch sichergestellt werden, dass die Probe kontinuierlich umgerührt wird.

Außerdem ist es dem Fachmann klar, dass die konkreten Werte der Sauerstoffzehrung extrem temperaturabhängig sind. Die Probe muss daher bei dem neuen Verfahren entweder auf konstante Temperatur gehalten werden, was durch Durchführen des Verfahrens in einem Klimaschrank oder in einem Wasserbad realisiert werden kann, oder die Temperatur der Probe muss zusätzlich fortlaufend erfasst werden.

Bei dem neuen Verfahren kann aus dem Umfang der erfolgten Sauerstoffzufuhr und aus der Änderung des nach jeder Unterbrechung der Sauerstoffzufuhr bestimmten Sauerstoffgehalts der Probe ein erforderlicher Restbehandlungsumfang für den Schlamm bestimmt werden. Insbesondere kann bestimmt werden, wie viel Sauerstoff dem Schlamm, von dem die Probe stammt, noch zugeführt werden muss, bis er hinreichend stabilisiert ist. Daneben lässt die Analyse der Änderung des nach jeder Unterbrechung der Sauerstoffzufuhr bestimmten Sauerstoffgehalts der Probe auch eine Aussage darüber zu, wie weit der Schlamm, von dem die Probe stammt, von einer weitergehenden Sauerstoffzufuhr noch profitieren könnte, auch wenn er bereits keine übermäßige Sauerstoffzehrung mehr aufweist.

Auch während der Unterbrechungen der Sauerstoffzufuhr sollte die Probe kontinuierlich umgerührt werden, um einheitliche Messbedingungen für den Sauerstoffgehalt der Probe bereitzustellen. Auf diese Weise wird auch für eine gleichmäßige Anströmung einer Sauerstoffsonde in der Probe gesorgt.

Bei einer konkreten Anwendung des neuen Verfahrens werden für jede Klärschlammenge, die in ein Vererdungsbecken (siehe z.B. http://www.eko-plant.de) eingeleitet wird, die nach jeder Unterbrechung der Sauerstoffzufuhr ermittelten Sauerstoffgehalte der Probe oder die aus der Änderung des Sauerstoffgehalts bestimmte metabolische Stabilität an eine Überwachungseinrichtung für eine Langzeitüberwachung der Belastung des Vererdungsbeckens übermittelt. Welche Belastungen Pflanzen in einem Vererdungsbecken ausgesetzt sind, ist streng genommen nur durch eine vollkommene Analyse der in ein Vererdungsbecken eingeleiteten Substanzen zu erfassen. Es stellt sich jedoch heraus, dass die Überwachung der metabolischen Stabilität der in das Vererdungsbecken eingeführten Klärschlämme die Belastung der Pflanzen in einem Vererdungsbecken bereits weitgehend erfasst. Dies gilt in zunehmendem Maße, wenn an der bei dem erfindungsgemäßen Verfahren genommenen Probe zusätzlich eine Redoxmessung, eine Ammoniummessung und/oder eine Nitratmessung vorgenommen wird/werden. Diese Messungen können an der jeweiligen Probe einmal oder wie die Messung des Sauerstoffgehalts auch in Abhängigkeit einer zusätzlichen Sauerstoffzufuhr zu der Probe durchgeführt werden.

Durch die rechtzeitige Untersuchung der in ein Vererdungsbecken einzubringenden Klärschlämme, die nach dem Abziehen aus der Nachklärung einer Kläranlage z.B. in einem Stapelbehälter gesammelt werden, ist es mit dem erfindungsgemäßen Verfahren möglich, eine schnelle Entscheidung über die Eignung des aktuell vorliegenden Klärschlamms zu treffen. Wenn die aktuell bestimmte metabolische Stabilität für den Vererdungszweck noch nicht ausreichend ist, kann die Beschickung des Vererdungsbeckens mit diesem Schlamm ausgesetzt und der Klärschlamm z.B. einer Nachbelüftung zugeführt werden, bis er von seiner Stabilität her für eine Verwendung auf dem Vererdungsbecken geeignet ist. Dadurch wird einer möglichen Schädigung der Pflanzen aufgrund einer nicht ausreichenden Schlammqualität vorgebeugt.

Die mittels des erfindungsgemäßen Verfahrens ermittelten Kennwerte zur aktuellen Stabilität eines Schlamms können auch mit zuvor ermittelten Soll-Werten verglichen werden, die eine ausreichende Stabilisierung kennzeichnen. Entsprechen die Ist-Werte nicht den vorgegebenen Soll-Werten, muss weiterbelüftet werden. Dieser Vorgang lässt sich weitestgehend automatisieren, z.B. durch einen zentralen Rechner, der sowohl die Ermittlung der Stabilitätskennwerte aus den Sauerstoffmesswerten durchführt als auch den Vergleich Ist-SollWert vornimmt als auch die Durchführung einer eventuellen Weiterbelüftung steuert als auch letztlich eine Beschickungsfreigabe für ein Vererdungsbeet erteilt.

Vorteilhafte Weiterbildungen der Erfindung ergeben sich aus den abhängigen Patentansprüchen und der gesamten Beschreibung. Weitere Merkmale sind den Zeichnungen - insbesondere den dargestellten Geometrien und den relativen Abmessungen mehrerer Bauteile zueinander sowie deren relativer Anordnung und Wirkverbindung - zu entnehmen. Die Kombination von Merkmalen unterschiedlicher Ausführungsformen der Erfindung oder von Merkmalen unterschiedlicher Patentansprüche abweichend von den gewählten Rückbeziehungen ist ebenfalls möglich und wird hiermit angeregt. Dies betrifft auch solche Merkmale, die in separaten Zeichnungsfiguren dargestellt sind oder bei deren Beschreibung genannt werden. Diese Merkmale können auch mit Merkmalen unterschiedlicher Patentansprüche kombiniert werden.

### KURZBESCHREIBUNG DER FIGUREN

Die Erfindung wird im Folgenden anhand eines bevorzugten Ausführungsbeispiels unter Bezugnahme auf die beigefügten Figuren weiter erläutert und beschrieben.
- **Fig. 1**: zeigt eine Messanordnung zur Durchführung des neuen Verfahrens.
- **Fig. 2**: zeigt eine Auftragung des Sauerstoffgehalts und der Temperatur einer Probe eines Schlamms bei einer Durchführung des neuen Verfahrens.
- **Fig. 3**: zeigt eine Auftragung von Atmungsraten, die aus den in Fig. 2 aufgetragenen Daten abgeleitet wurden; und
- **Fig. 4**: zeigt die aus einer anderen Durchführung des neuen Verfahrens abgeleiteten Werte für den überschüssigen gelösten Sauerstoff in einer Probe.

### FIGURENBESCHREIBUNG

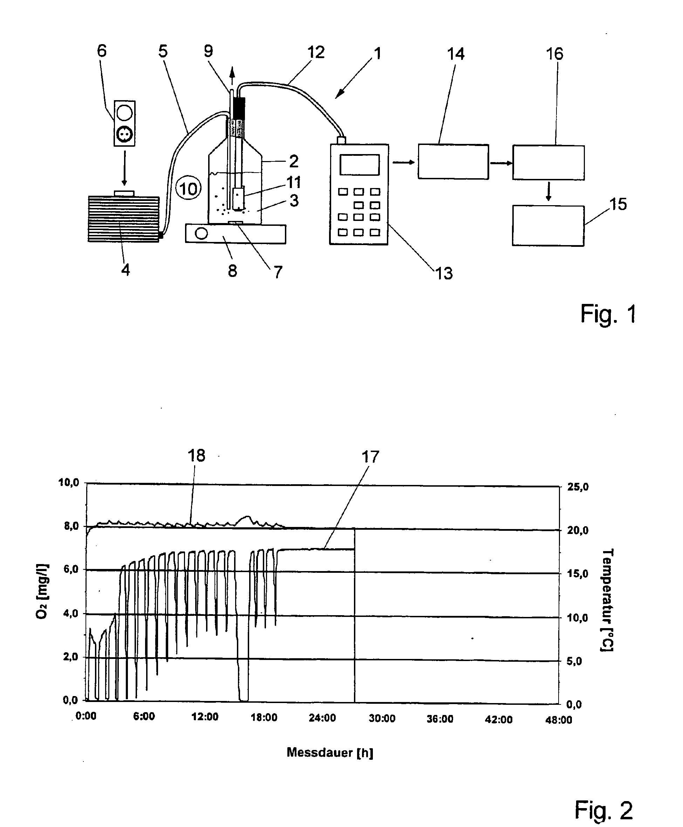

**Fig. 1** zeigt eine Vorrichtung 1 zur Bestimmung der metabolischen Stabilität von Schlamm, insbesondere Klärschlamm. Hierzu nimmt eine Winklerflasche 2 eine Probe 3 des Schlamms auf. In die Probe 3 ragt ein von einer Aquariumpumpe 4 kommender Schlauch 5 hinein, um Luft in die Probe 3 einzublasen und der Probe 3 auf diese Weise Sauerstoff zuzuführen. Die Aquariumpumpe 4 wird mit einer Zeitschaltuhr 6 in Intervallen ein- und ausgeschaltet, die in Zusammenhang mit Fig. 2 noch näher erläutert werden werden. Um den der Probe 3 zugeführten Sauerstoff gleichmäßig in der Probe zu verteilen, ist ein Rührfisch 7 in der Winklerflasche 2 vorgesehen, der von einem Magnetrührer 8 angetrieben wird. Der die Probe 3 umrührende Rührfisch 7 verhindert auch ein Absetzen von Feststoffen der Probe 3 am Boden der Winklerflasche 2, d. h. eine Phasenseparation in der Probe 3. Von der Probe 3 nicht absorbierte Luft gelangt aus dem Hals der Winklerflasche 2 in die Umgebung. Dies ist hier durch ein Abluftröhrchen 9 angedeutet. Der Innenraum der Winklerflasche 2 befindet sich immer auf demselben Druck wie die Umgebung 10 der Winklerflasche 2. Das Abluftröhrchen 9 lässt jedoch keine nennenswerten Anteile von Luft und insbesondere Sauerstoff aus der Umgebung 10 in die Winklerflasche 2 eintreten. Durch den Hals der Winklerflasche 2 ragt neben dem Schlauch 5 eine Sauerstoffsonde 11 in die Probe 3 hinein. Die Sauerstoffsonde 11 umfasst auch einen hier nicht separat wiedergegebenen Temperaturfühler. Die Sauerstoffsonde 11 ist über eine Anschlussleitung 12 an ein Sauerstoffmessgerät 13 angeschlossen, das zum Ermitteln des aktuellen Sauerstoffgehalts und der Temperatur der Probe 3 dient. Das Sauerstoffmessgerät 13 ist hier an einen Wandler 14 angeschlossen, der die Messwerte des Sauerstoffmessgeräts 13 für die Übertragung an einen Rechner 15 über eine Datenübertragungsstrecke 16 umwandelt. Die Datenübertragungsstrecke 16 kann ein Datenfernübertragungsnetz, ein Intranet oder dergleichen sein. Die Winklerflasche 2 kann, was hier nicht dargestellt ist, zur Konstanthaltung der Temperatur der Probe 3 in einem Wasserbad oder einem Klimaschrank 3 angeordnet sein. Außerdem können weitere Messungen neben derjenigen des Sauerstoffgehalts und der Temperatur an der Probe 3 vorgenommen werden. Primär geht es jedoch bei der Vorrichtung 1 um die Bestimmung der metabolischen Stabilität von Schlamm, von dem die Probe 3 stammt, durch Bestimmung der Sauerstoffzehrung der Probe. Zu diesem Zweck wird die Probe 3 über den Schlauch 5 durch die Aquariumpumpe 4 intermittierend belüftet. Einem Zeitraum von 45 min. mit Belüftung, in dem die Aquariumpumpe 4 durch die Zeitschaltuhr 6 eingeschaltet ist, folgt ein Zeitraum von 15 min. ohne Belüftung, in dem die Aquariumpumpe 4 durch die Zeitschaltuhr 6 ausgeschaltet ist. Hieran schließt sich wieder ein Zeitraum mit Belüftung an, auf den ein weiterer Zeitraum ohne Belüftung folgt usw.

Die gesamte Messdauer sollte mindestens 10 h, besser 24 h oder gar 48 h betragen. Während der Phasen der Belüftung sollte der Probe 3 Sauerstoff im Überschuss zugeführt werden, so dass der Sauerstoffgehalt in der Probe 3 seine Sättigungskonzentration von typischerweise ≥ 6 mg Sauerstoff/Liter Probe erreicht. Dies wird durch die Messung des Sauerstoffgehalts in der Probe 3 während der erfolgenden Sauerstoffzuguhr überwacht. Die Erfassung des Sauerstoffgehalts der Probe 3 erfolgt dabei vorzugsweise nicht kontinuierlich sondern in gleichmäßigen Abständen, insbesondere im Minutenabstand. Gleichzeitig wird die Temperatur der Probe 3 überwacht, weil unterschiedliche Temperaturen der Probe 3 zu unterschiedlichen Löslichkeiten von Sauerstoff und biologischen Aktivitäten in der Probe 3 führen. Bei unterschiedlichen Temperaturen gemessene Sauerstoffgehalte sollten daher auf eine einheitliche Temperatur, wie beispielsweise 20 °C (Raumtemperatur) umgerechnet werden. Hierauf kann verzichtet werden, wenn die Temperatur der Probe 3 durch ein Wasserbad oder ein Klimaschrank in engen Grenzen konstant gehalten wird.

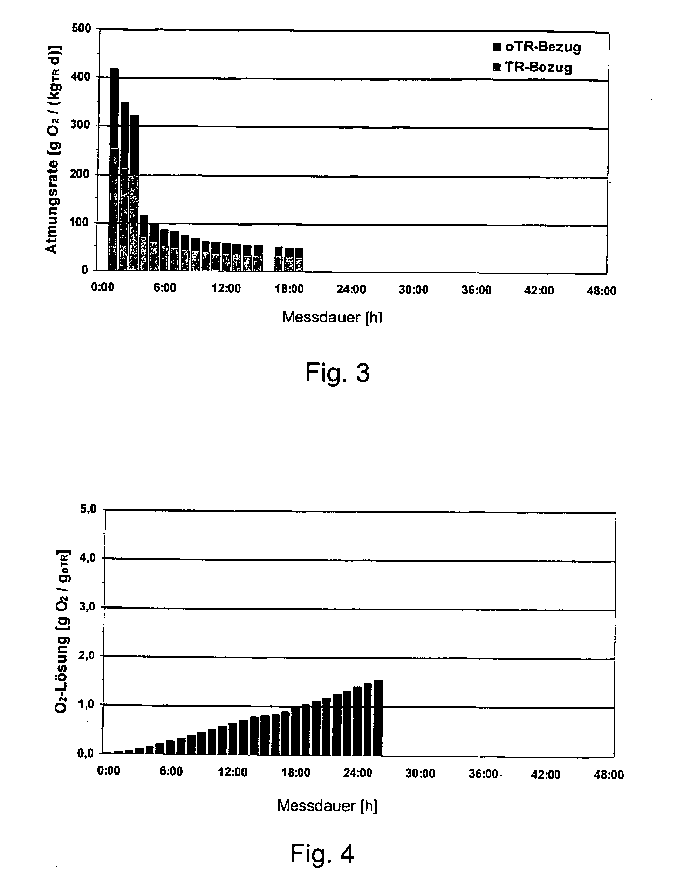

Wenn die Sauerstoffzufuhr zu der Probe 3 unterbrochen wird, fällt der Sauerstoffgehalt in der Probe 3 durch die Sauerstoffatmung der Probe 3, die hier auch als Sauerstoffzehrung bezeichnet wird, ab. Durch den mit dem Sauerstoffmessgerät 13 erfassten Verlauf des Sauerstoffgehalts in der Probe 3 nach Abschalten der Aquariumpumpe 4 kann der aktuelle Wert der Sauerstoffatmung der Probe 3 bestimmt werden. Hierauf wird im Zusammenhang mit Fig. 3 noch näher eingegangen werden. Außerdem ist es möglich, den in der Probe kumulierten überschüssigen, d. h. nicht verbrauchten gelösten Sauerstoff zu ermitteln. Während eine noch große Atmungsrate ein Hinweis darauf ist, dass der Schlamm, von dem die Probe 3 stammt, noch nicht stabilisiert ist, weist eine größere Konzentration an kumuliertem überschüssigen gelösten Sauerstoff auf eine höhere Stabilisierung des Schlamms hin. Aus dem Umfang der Sauerstoffzufuhr bis zum Erreichen von wünschenswerten Werten der Atmungsrate und des in der Probe gelösten unverbrauchten Sauerstoffs kann auf den Umfang einer notwendigen Nachbehandlung des Schlamms geschlossen werden, von dem die Probe 3 stammt. Dabei ist das relevante Maß für die Sauerstoffzufuhr die Zeitdauer des Betriebs der Aquariumpumpe 4. Weil der Sauerstoff jeweils im Überschuss zugeführt wird, kommt es auf die konkrete Menge des der Probe von der Aquariumpumpe mit der in die Probe 3 eingeblasenen Luft zugeführten Sauerstoffs nicht an.

**Fig. 2** zeigt mit einer Kurve 17 den Verlauf des Sauerstoffgehalts, d. h. der Sauerstoffkonzentration in mg/l in der Probe 3 über der Messdauer in Stunden. Weiterhin ist mit einer Kurve 18 der Verlauf der Temperatur über der Messdauer aufgetragen. Die Temperatur liegt nahezu konstant bei wenig über 20 °C. Der Sauerstoffgehalt in der Probe steigt während jedes Intervalls, in dem die Probe belüftet wird (außer während des ersten Intervalls, was auf eine Temperaturerhöhung der Probe in diesem Zeitraum zurückzuführen sein mag) an und erreicht ab dem vierten Intervall der Belüftung einen Wert oberhalb 6 mg/l, d. h. die Sättigungskonzentration von Sauerstoff in der Probe. Während der Intervalle, in denen die Sauerstoffzufuhr zu der Probe 3 unterbrochen wird, fällt der Sauerstoffgehalt in der Probe 3 ab. Anfangs geht er während jedes Intervalls auf nahezu null zurück. Nach etwa 12 h fällt der Sauerstoffgehalt in den 15-minütigen Unterbrechungen der Sauerstoffzufuhr nur noch auf einen Wert oberhalb 3 mg/l. Dies ist jedoch noch nicht der Wert an kumuliertem überschüssigen gelösten Sauerstoff in der Probe, der sich erst über einen längeren Zeitraum einstellt, der aber dennoch aus den während des jeweiligen Intervalls der Unterbrechung der Sauerstoffzufuhr bestimmten Sauerstoffgehalten ermittelt werden kann.

Dies wird durch das eine Intervall vor 18 h Messdauer dokumentiert, in dem die Aquariumpumpe 4 von der Zeitschaltuhr 6 nicht wieder eingeschaltet wurde und entsprechend der Sauerstoffgehalt in der Probe über einen längeren Zeitraum weiter abfallen konnte.

In **Fig. 3** ist die Atmungsrate der Probe über der Messdauer aufgetragen, die aus dem Verlauf des Sauerstoffgehalts der Probe während der Unterbrechungen der Sauerstoffzufuhr zu der Probe bestimmt wurde. Dabei sind die Werte einmal auf den Trockenrückstand (TR) der Probe und einmal auf den organischen Trockenrückstand (oTR) der Probe bezogen. Die Atmungsrate der Probe fällt über die Messdauer von sehr hohen Werten auf Werte von unter 60 g Sauerstoff/kg TR bzw. 100 g Sauerstoff/kg oTR binnen etwa 5 h ab. Dies bedeutet, dass der Schlamm, von dem die Probe 3 stammt, mit einer zusätzlichen Sauerstoffbehandlung, die der Sauerstoffzufuhr während der ersten 5 h der Messdauer entspräche, hinreichend stabilisiert werden könnte aber im Moment noch nicht hinreichend stabilisiert ist. Der weitere Verlauf der Atmungsrate mit höherer Messdauer zeigt das Potential an, wie weit der Schlamm durch eine darüber hinausgehende Sauerstoffbehandlung noch stabilisiert werden könnte.

**Fig. 4** zeigt den bei einer entsprechenden Probe über eine längere Messdauer bestimmten kumulierten überschüssigen, d. h. nicht verbrauchten gelösten Sauerstoff in g/g oTR. Dieser Wert steigt mit der Messdauer und der damit einhergehenden Sauerstoffzufuhr zu der Probe kontinuierlich an. Auch das Maß dieses kumulierten Sauerstoffgehalts kann als Maß für die metabolische Stabilität des Schlamms, von dem die Probe stammt, oder für die bei diesem Schlamm mit bestimmter Sauerstoffzufuhr erreichbare metabolische Stabilität verwendet werden.

### BEZUGSZEICHENLISTE

- 1: Vorrichtung
- 2: Winklerflasche
- 3: Probe
- 4: Aquariumpumpe
- 5: Schlauch
- 6: Zeitschaltuhr
- 7: Rührfisch
- 8: Magnetführer
- 9: Abluftröhrchen
- 10: Umgebung
- 11: Sauerstoffsonde
- 12: Anschlussleitung
- 13: Sauerstoffmessgerät
- 14: Wandler
- 15: Rechner
- 16: Datenübertragungsstrecke
- 17: Kurve
- 18: Kurve

## Patentansprüche

1. Verfahren zur Bestimmung der metabolischen Stabilität von Schlamm, insbesondere Klärschlamm, wobei eine Sauerstoffzehrung des Schlamms nach Unterbrechung einer Sauerstoffzufuhr zu dem Schlamm beobachtet wird, **dadurch gekennzeichnet, dass** die Sauerstoffzufuhr zu einer von dem Schlammgenommenen Probe in gleichen zeitlichen Abständen mindestens siebenmal hintereinander unterbrochen wird, dass mindestens einmal während jeder Unterbrechung der Sauerstoffzufuhr in gleichem Abstand zu dem Beginn der Unterbrechung der Sauerstoffzufuhr ein Sauerstoffgehalt der Probe ermittelt wird und dass die metabolische Stabilität unter Berücksichtigung der Änderung des nach jeder Unterbrechung der Sauerstoffzufuhr ermittelten Sauerstoffgehalts bestimmt wird.

2. Verfahren nach Anspruch 1 , **dadurch gekennzeichnet, dass** während jeder Unterbrechung der Sauerstoffzufuhr in gleichen Abständen von dem Beginn der Unterbrechung der Sauerstoffzufuhr der Sauerstoffgehalt der Probe mehrfach ermittelt und der Verlauf des Sauerstoffgehalts während jeder Unterbrechung der Sauerstoffzufuhr beobachtet wird.

3. Verfahren nach einem der Ansprüche 1 und 2, **dadurch gekennzeichnet, dass** mindestens einmal vor jeder Unterbrechung der Sauerstoffzufuhr der Sauerstoffgehalt der Probe ermittelt wird.

4. Verfahren nach Anspruch 1, **dadurch gekennzeichnet, dass** vor jeder Unterbrechung der Sauerstoffzufuhr in gleichen Abständen von dem Beginn der Sauerstoffzufuhr der Sauerstoffgehalt der Probe mehrfach ermittelt und der Verlauf des Sauerstoffgehalts während der Sauerstoffzufuhr beobachtet wird.

5. Verfahren nach einem der Ansprüche 1 bis 4, **dadurch gekennzeichnet, dass** die Sauerstoffzufuhr vor jeder Unterbrechung für mindestens 30 Minuten andauert und dass jede Unterbrechung der Sauerstoffzufuhr mindestens 10 Minuten andauert.

6. Verfahren nach einem der Ansprüche 1 bis 5, **dadurch gekennzeichnet, dass** die Sauerstoffzufuhr den Sauerstoffgehalt der Probe bis auf ihre Sättigungskonzentration anhebt.

7. Verfahren nach einem der Ansprüche 1 bis 6, **dadurch gekennzeichnet, dass** die Sauerstoffzufuhr zu der Probe durch Einblasen von Druckluft bewirkt wird.

8. Verfahren nach einem der Ansprüche 1 bis 7, **dadurch gekennzeichnet, dass** aus dem Umfang der Sauerstoffzufuhr und aus der Änderung des nach jeder Unterbrechung der Sauerstoffzufuhr bestimmten Sauerstoffgehalts der Probe ein erforderlicher Restbehandlungsumfang für den Schlamm bestimmt wird.

9. Verfahren nach einem der Ansprüche 1 bis 8, **dadurch gekennzeichnet, dass** für jede Klärschlammmenge, die in ein Vererdungsbecken eingeleitet wird, die nach jeder Unterbrechung der Sauerstoffzufuhr ermittelten Sauerstoffgehalte der Probe oder die aus der Änderung des Sauerstoffgehalts bestimmte metabolische Stabilität an eine Überwachungseinrichtung für eine Langzeitüberwachung der Belastung und/oder Steuerung der Beschickung des Vererdungsbecken übermittelt werden.

## Claims

1. A method of determining the metabolic stability of sludge, particularly of digested sludge, the oxygen consumption of the sludge after an interruption of an oxygen supply to the sludge being observed, **characterized in that** the oxygen supply to a sample taken from the sludge is interrupted at least seven consecutive times at equal intervals of time, that at least once during each interruption of the oxygen supply an oxygen content of the sample is determined at an equal distance to the beginning of the interruption of the oxygen supply, and that the metabolic stability is determined taking into account the change of the oxygen content determined after each interruption of the oxygen supply.

2. The method of claim 1, **characterized in that** the oxygen content of the sample is determined several times during each interruption of the oxygen supply at equal distances from the beginning of the interruption of the oxygen supply, and that the course of the oxygen content is observed during each interruption of the oxygen supply.

3. The method of any of the claims 1 and 2, **characterized in that** the oxygen content of the sample is determined at least once prior to each interruption of the oxygen supply.

4. The method of claim 1, **characterized in that** the oxygen content of the sample is determined several times prior to each interruption of the oxygen supply at equal distances to the beginning of the oxygen supply, and that the course of the oxygen content during the oxygen supply is observed.

5. The method of any of the claims 1 to 4, **characterized in that** the oxygen supply prior to each interruption continues for at least 30 minutes, and that each interruption of the oxygen supply continues for at least 10 minutes.

6. The method of any of the claims 1 to 5, **characterized in that** the oxygen supply raises the oxygen content of the sample up to its saturation concentration.

7. The method of any of the claims 1 to 6, **characterized in that** the oxygen supply to the sample is effected by blowing in pressurized air.

8. The method of any of the claims 1 and 7, **characterized in that** a necessary remaining treatment extent of the sludge is determined from the extent of the oxygen supply and from the change of the oxygen content of the sample determined after each interruption of the oxygen supply.

9. The method of any of the claims 1 to 8, **characterized in that**, for each amount of digested sludge which is discharged in a transformation into humus basin, the oxygen contents of the sample determined after each interruption of the oxygen supply or the metabolic stability determined from the change of the oxygen content is communicated to a surveying device for a long term surveillance of the load and/or for controlling the loading of the transformation into humus basin.

## Revendications

1. Procédé pour la détermination de la stabilité métabolique d'une boue, en particulier d'une boue de décantation, dans lequel on observe une consommation d'oxygène de la boue après l'interruption d'une amenée d'oxygène à la boue, **caractérisé en ce que** l'amenée d'oxygène à un échantillon prélevé dans la boue est interrompue à intervalles identiques au moins sept fois à la suite, **en ce que**, au moins une fois pendant chaque interruption de l'amenée d'oxygène, une teneur en oxygène de l'échantillon est déterminée à intervalles identiques au début de l'interruption de l'amenée d'oxygène et **en ce que** la stabilité métabolique est déterminée en tenant compte de la variation de la teneur en oxygène déterminée après chaque interruption de l'amenée d'oxygène.

2. Procédé selon la revendication 1, **caractérisé en ce que**, pendant chaque interruption de l'amenée d'oxygène, la teneur en oxygène de l'échantillon est déterminée plusieurs fois à intervalles identiques à partir du début de l'interruption de l'amenée d'oxygène et la variation de la teneur en oxygène est observée pendant chaque interruption de l'amenée d'oxygène.

3. Procédé selon l'une quelconque des revendications 1 et 2, **caractérisé en ce que** la teneur en oxygène de l'échantillon est déterminée au moins une fois avant chaque interruption de l'amenée d'oxygène.

4. Procédé selon la revendication 1, **caractérisé en ce que**, avant chaque interruption de l'amenée d'oxygène, la teneur en oxygène de l'échantillon est déterminée plusieurs fois à intervalles identiques à partir du début de l'amenée d'oxygène et la variation de la teneur en oxygène est observée pendant l'amenée d'oxygène.

5. Procédé selon l'une quelconque des revendications 1 à 4, **caractérisé en ce que** l'amenée d'oxygène avant chaque interruption dure au moins 30 minutes et **en ce que** chaque interruption de l'amenée d'oxygène dure au moins 10 minutes.

6. Procédé selon l'une quelconque des revendications 1 à 5, **caractérisé en ce que** l'amenée d'oxygène élève la teneur en oxygène de l'échantillon jusqu'à sa concentration de saturation.

7. Procédé selon l'une quelconque des revendications 1 à 6, **caractérisé en ce que** l'amenée d'oxygène à l'échantillon est réalisée par soufflage d'air comprimé.

8. Procédé selon l'une quelconque des revendications 1 à 7, **caractérisé en ce qu'**un volume de traitement résiduel est déterminé pour la boue à partir du volume de l'amenée d'oxygène et à partir de la variation de la teneur en oxygène de l'échantillon déterminée après chaque interruption de l'amenée d'oxygène.

9. Procédé selon l'une quelconque des revendications 1 à 8, **caractérisé en ce que**, pour chaque quantité de boue de décantation qui est introduite dans un bassin d'enfouissement, les teneurs en oxygène de l'échantillon déterminées après chaque interruption de l'amenée d'oxygène ou la stabilité métabolique déterminée à partir de la variation de la teneur en oxygène sont transmises à un dispositif de surveillance pour une surveillance prolongée de la charge et/ou pour une régulation du chargement du bassin d'enfouissement.
